# EUROPEAN PATENT SPECIFICATION

(11) **EP 3 366 282 B1**
(45) Date of publication and mention of the grant of the patent: **01.10.2025**
(21) Application number: 18158766.8
(22) Date of filing: 27.02.2018
(51) Int. Cl.: A61K 9/20, A61K 31/55

(54) **SOLID ORAL PHARMACEUTICAL COMPOSITIONS OF IVABRADINE**
FESTE ORALE PHARMAZEUTISCHE ZUSAMMENSETZUNGEN VON IVABRADIN
COMPOSITIONS PHARMACEUTIQUES ORALES SOLIDES D'IVABRADINE

(30) Priority: 28.02.2017 TR 201703066; 26.02.2018 TR 201802737
(43) Date of publication of application: 29.08.2018
(73) Proprietor: Sanovel Ilac Sanayi ve Ticaret A.S., Sisli - Istanbul (TR)
(72) Inventor: TÜRKYILMAZ, Ali, 34460 Istanbul (TR); PALANTÖKEN, Arzu, 34460 Istanbul (TR); GÜNER, Dicle, 34460 Istanbul (TR)
(74) Representative: Genç Ilhan, Oznur

(56) References cited:
- WO-A1-2015/001133
- WO-A1-2016/198154
- WO-A2-2011/098582

## Description

### Field of the invention

The present invention relates to solid oral pharmaceutical compositions, comprising ivabradine hydrochloride or pharmaceutically acceptable polymorphs thereof and at least one cellulose derivative excipient.

### Background of the invention

Ivabradine, (marketed under the trade name Corlanor) is a medication used for the symptomatic management of stable heart related chest pain and heart failure not fully managed by beta blockers.

The chemical name of ivabradine is 3-{3-[{[(7S)-3,4-dimethoxybicyclo[4.2.0]octa-1,3,5-trien-7-yl]methyl}(methyl)amino]propyl}-7,8-dimethoxy-1,3,4,5-tetrahydro-2H-3-benzazepin-2-one and its chemical structure is shown in the Formula 1.

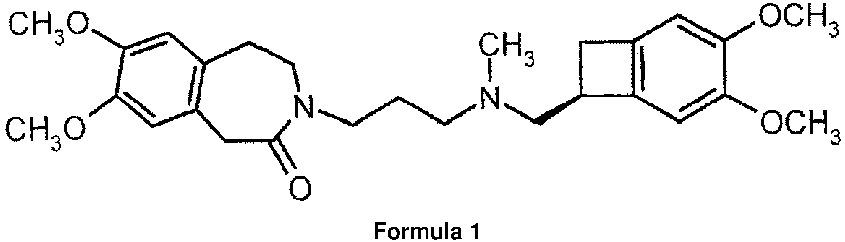

Ivabradine is a heart rate lowering agent. It acts by selective and specific inhibition of the cardiac pacemaker If current, an important ionic current that usually controls spontaneous diastolic depolarization in the sinus node and thereby regulates heart rate.

Ivabradine molecule is disclosed in EP0534859. Ivabradine hydrochloride exhibits polymorphism. WO2006092493 discloses polymorph β of ivabradine hydrochloride, its process of preparation and compositions comprising this polymorph. Polymorph β is the most stable form and is present in the marketed product. Other polymorphic forms of ivabradine hydrochloride are disclosed in WO2005110993, WO2006092491, WO2006092492, WO2006092494, WO2007042656, WO2007042657 and WO2013064307. It's known from the prior art, ivabradine hydrochloride crystallizes easily and polymorphic transitions of ivabradine hydrochloride take place rather easily, especially in drug products.

Amorphous ivabradine hydrochloride and methods for its preparation are disclosed in WO2008146308, CN101597261 and CN101463008.

EP1345594 describes thermoformable solid pharmaceutical compositions for controlled release of ivabradine by using polymethacrylates polymers.

Ivabradine has content uniformity problem which is a pharmaceutical analysis parameter for the quality control of capsules or tablets. In this present invention, these problems are also solved by adjusting the ratio of excipients. Accordingly, there is a need for an improved pharmaceutical composition of ivabradine or pharmaceutically acceptable salt to achieve desired dissolution profile and release kinetic while overcoming stability and content uniformity problems.

Ivabradine is an active agent which has also stability problems. Therefore, it is necessary to avoid degradation and polymorphic transition of ivabradine over the shelf life of the product. Mechanical stress associated with process and using high amount of excipients lead to polymorphic transformation of ivabradine. Therefore, it is important to use excipients in a specific amount that decrease the degradation and sustain the stability.

Different polymorphs can lead to differences in solubility, dissolution profiles, and therefore bioavailability and stability of the product. Since ivabradine hydrochloride exhibits polymorphic transitions easily, it is a challenge to formulate a stable composition comprising ivabradine hydrochloride. Stable products should not undergo polymorphic transformation during their production and shelf life.

WO 2016/198154 A discloses pharmaceutical formulations of solid dosage forms for oral administration comprising ivabradine HCl polymorph IV or δ. The main concern of WO 2016/198154 A is the selection of the optimal combination of pharmaceutical acceptable excipients and method of preparation in order to achieve the appropriate dissolution profile and stability for the finished dosage form. Accordingly, low or non-hygroscopic anhydrous excipients are suggested within WO 2016/198154 A in order to avoid polymorphic transformation of ivabradine. Dimethicone and Syloid ^{®} FP (specific type of colloidal silicon dioxide) are also cited as suitable for adsorbing moisture.

The present invention is based on the findings that stable pharmaceutical compositions and dosage forms comprising ivabradine hydrochloride having improved content uniformity and/or flowability and/or dissolution by using a microcystalline cellulose excipient.

### Detailed description of the Invention

The main object of the present invention is to provide solid oral pharmaceutical compositions comprising ivabradine or a pharmaceutically acceptable salt thereof or pharmaceutically acceptable polymorphs thereof, having a content uniformity with high stability and bioavailability, wherein the pharmaceutically acceptable salt is ivabradine hydrochloride.

This object is solved by a solid oral pharmaceutical composition comprising ivabradine hydrochloride and microcrystalline cellulose wherein ivabradine hydrochloride has a polymorph form selected from the group consisting of form S, form II, form IV, form delta and form delta D; wherein said solid oral pharmaceutical composition further comprises lactose and the weight ratio of microcrystalline cellulose to lactose is more than 1.00.

In one embodiment, the solid oral composition comprises ivabradine hydrochloride or pharmaceutically acceptable polymorphs thereof in an amount of 0.01% to 20.00% by weight of the total composition, preferably 1.00% to 10.00% and more preferably 5.00% to 10.00%.

The cellulose derivative excipient is microcrystalline cellulose.

According to these embodiments, the amount of microcrystalline cellulose is between 30.00% and 70.00% by weight of the composition, preferably between 40.00% and 60.00%, more preferably between 40.00% and 55.00%.

Using microcrystalline cellulose in the composition, especially in these ratios, provides a uniform formulation content which is a challenge in the tablet formulations of ivabradine hydrochloride or pharmaceutically acceptable polymorphs thereof.

According to this embodiment, the composition is in the form of coated tablet, trilayer tablet, bilayer tablet, multilayer tablet, orally disintegrating tablet, mini tablet, pellet, sugar pellet, buccal tablet, sublingual tablet, effervescent tablet, immediate release tablet, modified release tablet, film-coated tablet, gastric disintegrating tablet, pill, capsule, oral granule, powder, coated bead system, microsphere, tablet in tablet, inlay tablet, dragee, sachet, orally administrable film.

In a preferred embodiment, the composition is in the form of a film-coated tablet.

According to this embodiment, the film-coating has a thickness of 1 to 120 µm. Preferably the film-coating has a thickness of 50 to 110 µm.

It has been surprisingly found that using the film coating in a specific thickness takes part in enhancing the stability of the composition comprising ivabradine hydrochloride or pharmaceutically acceptable polymorphs thereof.

Thickness of the film coating is measured by using conventional methods.

Suitable film-coating layer may also preferably be used for the protection from the moisture, light and oxygen. It can be selected from the group comprising polyvinyl alcohol-polyethylene glycol copolymers (Kollicoat IR); polyvinyl alcohol or copolymers or mixtures thereof (Opadry AMB II); polyvinylpyrrolidone-vinyl acetate copolymers (Kollidon VA 64); ethylcellulose dispersions (Surelease); polyethylene glycol; polyvinyl alcohol and lecithin (Sheffcoat PVA+); polyvinylpyrrolidone and all kinds of OpadryTM, as well as pigments, dyes, titanium dioxide, iron oxide, talc or polymethylmetacrylate copolymers (Eudragit).

According to one embodiment, the composition further comprises at least one pharmaceutically acceptable excipient which is selected from a group comprising fillers, lubricants, disintegrants, glidants or mixtures thereof.

Suitable fillers are selected from a group comprising lactose, microcrystalline cellulose, dibasic calcium phosphate, mannitol, spray-dried mannitol, starch, dextrose, sucrose, fructose, maltose, sorbitol, xylitol, inositol, kaolin, inorganic salts, polysaccharides, sodium chloride, dextrates, lactitol, maltodextrin, sucrose-maltodextrin mixture, trehalose, sodium carbonate, sodium bicarbonate, calcium carbonate or mixtures thereof.

In one preferred embodiment, the filler is lactose.

In one embodiment, the amount of lactose is between 20.00% and 50.00% by weight of the composition, preferably between 20.00% and 45.00%, more preferably between 20.00% and 40.00%.

Particle size distribution of the filler plays a significant role for the qualification of the composition subjected to the invention. As used herein, 'particle size distribution' means the cumulative volume size distribution as tested by any conventionally accepted method such as the laser diffraction method (Malvern analysis).

Laser diffraction measures particle size distributions by measuring the angular variation in intensity of light scattered as a laser beam passes through a dispersed particulate sample. Large particles scatter light at small angles relative to the laser beam and small particles scatter light at large angles, as illustrated below. The angular scattering intensity data is then analyzed to calculate the size of the particles responsible for creating the scattering. The particle size is reported as a volume equivalent sphere diameter.

According to this measuring method, the term D10 means, the size at which 10% by volume of the particles are finer and D50 means the size at which 50% by volume of the particles are finer and D90 means the size at which %90 by volume of the particles are finer.

The D50 value of the filler is ranging between 1 to 200 µm. Preferably, this value is ranging between 60 to 190 µm.

The D90 value of the filler is ranging between 1 to 500 µm. Preferably this value is ranging between 100 to 375 µm.

The D10 value of the filler is ranging between 1 to 50 µm. Preferably this value is ranging between 20 to 40 µm.

In a preferred embodiment, the filler is lactose. The D90 value of lactose is ranging between 1 to 500 µm. Preferably this value is ranging between 300 to 400 µm. The D50 value of lactose is ranging between 1 to 200 µm. Preferably this value is ranging between 150 to 200 µm.

These specific selections of particle size distribution values, especially the D90 value range of the filler ensure the uniformity of the content of active substance of ivabradine-containing pharmaceutical compositions.

The weight ratio of microcrystalline cellulose to lactose is more than 1.00, preferably this ratio is between 1.00 and 2.75. The stability of the composition is provided by the help of these specific ratios.

Suitable disintegrants are selected from a group comprising cross-linked carboxymethyl cellulose (croscarmellose sodium), sodium starch glycolate, pregelatinized starch, microcrystalline cellulose, crospovidone (cross-linked polyvinyl pyrrolidone), povidone, poloxamer, low-substituted hydroxypropyl cellulose, starch, sodium carboxymethyl cellulose, calcium carboxymethyl cellulose, carboxymethyl cellulose, docusate sodium, polyacrylate potassium, sodium alginate, alginic acid, alginates, ion-exchange resins, sodium glycine carbonate, sodium lauryl sulphate or mixtures thereof.

In a preferred embodiment, the disintegrant is selected from cross-linked carboxymethyl cellulose (croscarmellose sodium), sodium starch glycolate or mixtures thereof.

According to these embodiments, the amount of the disintegrant is between 1.00% and 40.00% by weight of the composition. Preferably the amount of the disintegrant is between 5.00% and 20.00% by weight of the composition.

Suitable lubricants are selected from a group comprising sodium stearyl fumarate, magnesium stearate, calcium stearate, zinc stearate, talc, waxes, boric acid, hydrogenated vegetable oil, sodium chlorate, magnesium lauryl sulfate, sodium oleate, sodium acetate, sodium benzoate, polyethylene glycol, stearic acid, fatty acid, fumaric acid, glyceryl palmitostearate, sodium lauryl sulphate or mixtures thereof.

In one preferred embodiment, the lubricant is sodium stearyl fumarate.

Selection of a lubricant is important for providing a good content uniformity which is a problem for the active agent. When sodium stearyl fumarate is used in this composition, good content uniformity is obtained. Since sodium stearyl fumarate is less hydrophobic than magnesium stearate, it provides better stability. Sodium stearyl fumarate is also much better than magnesium stearate in terms of hardness and disintegration time of tablets. Due to hydrophilic property of sodium stearyl fumarate, the composition does not have the disadvantages of magnesium stearate in respect of flowability and dissolution.

According to these preferred embodiments, the amount of sodium stearyl fumarate is between 1.00% and 10.00%, preferably between 3.00% and 5.00% by weight of the composition. These specific ratios help to ensure content uniformity of the composition.

In one embodiment, the weight ratio of sodium stearyl fumarate to microcrystalline cellulose is between 0.01 and 0.50, preferably between 0.02 and 0.20.

Suitable glidants are selected from colloidal silicon dioxide, talc, aluminum silicate or mixtures thereof.

In a preferred embodiment, the glidant is colloidal silicon dioxide or talc.

In one preferred embodiment, the glidant is colloidal silicon dioxide.

According to these preferred embodiments, the amount of colloidal silicon dioxide is between 0.10% and 5.00%, preferably between 0.10% and 1.00%, more preferably between 0.50% and 0.80% by weight of the composition.

Suitable polymorphs of ivabradine hydrochloride are form S, form II, form IV, form delta and form delta D.

### Example 1: Film-coated tablet

| **Ingredients** | **Amount (%)** |
|---|---|
| Ivabradine hydrochloride or pharmaceutically acceptable polymorphs thereof | 0.01-20.00 |
| Microcrystalline cellulose | 30.00-70.00 |
| Lactose | 20.00-50.00 |
| Croscarmellose sodium | 1.00-40.00 |
| Colloidal silicon dioxide | 0.10-5.00 |
| Sodium stearyl fumarate | 1.00-10.00 |
| **Total tablet** | **100** |
| Opadry AMB II | 1.00-5.00 |
| **Total film-coated tablet** | **101.00-105.00** |

### Example 2: Film-coated tablet

| **Ingredients** | **Amount (%)** |
|---|---|
| Ivabradine hydrochloride or pharmaceutically acceptable polymorphs thereof | 5.00-10.00 |
| Microcrystalline cellulose | 40.00-55.00 |
| Lactose | 20.00-40.00 |
| Croscarmellose sodium | 5.00-20.00 |
| Colloidal silicon dioxide | 0.10-5.00 |
| Sodium stearyl fumarate | 3.00-5.00 |
| **Total tablet** | **100** |
| Opadry AMB II | 1.00-5.00 |
| **Total film-coated tablet** | **101.00-105.00** |

The pharmaceutical compositions mentioned above are prepared by following these steps:

### Process 1 (Direct compression):

a. Mixing ivabradine hydrochloride or pharmaceutically acceptable polymorphs thereof and colloidal silicon dioxide
b. Adding microcrystalline cellulose, lactose, croscarmellose sodium to the mixture and mixing
c. Adding sodium stearyl fumarate to this mixture and mixing
d. Compressing this mixture into tablets
e. Coating these tablets

### Process 2 (Dry granulation):

a. Mixing ivabradine hydrochloride or pharmaceutically acceptable polymorphs thereof, lactose, microcrystalline cellulose, croscarmellose sodium and colloidal silicon dioxide
b. Passing the mixture through a roller rompactor and sieving
c. Adding sodium stearyl fumarate to the obtained granules and mixing
d. Compressing the mixture into tablets
e. Coating these tablets

### Example 3: Film-coated tablet

| **Ingredients** | **Amount (%)** |
|---|---|
| Ivabradine hydrochloride or pharmaceutically acceptable polymorphs thereof | 0.01-20.00 |
| Microcrystalline cellulose | 30.00-70.00 |
| Lactose | 20.00-50.00 |
| Sodium starch glycolate | 1.00-40.00 |
| Colloidal silicon dioxide | 0.10-5.00 |
| Sodium stearyl fumarate | 1.00-10.00 |
| **Total tablet** | **100** |
| Opadry AMB II | 1.00-5.00 |
| **Total film-coated tablet** | **101.00-105.00** |

### Example 4: Film-coated tablet

| **Ingredients** | **Amount (%)** |
|---|---|
| Ivabradine hydrochloride or pharmaceutically acceptable polymorphs thereof | 5.00-10.00 |
| Microcrystalline cellulose | 40.00-55.00 |
| Lactose | 20.00-40.00 |
| Sodium starch glycolate | 5.00-20.00 |
| Colloidal silicon dioxide | 0.10-5.00 |
| Sodium stearyl fumarate | 3.00-5.00 |
| **Total tablet** | **100** |
| Opadry AMB II | 1.00-5.00 |
| **Total film-coated tablet** | **101.00-105.00** |

The pharmaceutical compositions mentioned above are prepared by following these steps:

### Process 1 (Direct compression):

a. Mixing ivabradine hydrochloride or pharmaceutically acceptable polymorphs thereof and colloidal silicon dioxide
b. Adding microcrystalline cellulose, lactose, sodium starch glycolate to the mixture and mixing
c. Adding sodium stearyl fumarate to this mixture and mixing
d. Compressing this mixture into tablets
e. Coating these tablets

### Process 2 (Dry granulation):

a. Mixing ivabradine hydrochloride or pharmaceutically acceptable polymorphs thereof, lactose, microcrystalline cellulose, sodium starch glycolate and colloidal silicon dioxide
b. Passing the mixture through a roller rompactor and sieving
c. Adding sodium stearyl fumarate to the obtained granules and mixing
d. Compressing the mixture into tablets
e. Coating these tablets

## Claims

1. A solid oral pharmaceutical composition comprising ivabradine hydrochloride and microcrystalline cellulose wherein ivabradine hydrochloride has a polymorph form selected from the group consisting of form S, form II, form IV, form delta and form delta D; wherein said solid oral pharmaceutical composition further comprises lactose and the weight ratio of microcrystalline cellulose to lactose is more than 1.00.

2. The solid oral pharmaceutical composition according to claim 1, wherein the composition comprising ivabradine hydrochloride in an amount of 0.01% to 20.00% by weight of the total composition, preferably 1.00% to 10.00% and more preferably 5.00% to 10.00%.

3. The solid oral pharmaceutical composition according to claim 1, wherein the amount of microcrystalline cellulose is between 30.00% and 70.00% by weight of the composition, preferably between 40.00% and 60.00%, more preferably between 40.00% and 55.00%.

4. The solid oral pharmaceutical composition according to claim 1 or 2, wherein the composition is in the form of coated tablet, trilayer tablet, bilayer tablet, multilayer tablet, orally disintegrating tablet, mini tablet, pellet, sugar pellet, buccal tablet, sublingual tablet, effervescent tablet, immediate release tablet, modified release tablet, film-coated tablet, gastric disintegrating tablet, pill, capsule, oral granule, powder, coated bead system, microsphere, tablet in tablet, inlay tablet, dragee, sachet or orally administrable film.

5. The solid oral pharmaceutical composition according to claim 4, wherein the composition is in the form of a film-coated tablet.

6. The solid oral pharmaceutical composition according to claim 5, wherein the film coating has a thickness of 1 to 120 µm.

7. The solid oral pharmaceutical composition according to claim 6, wherein the film coating selected from the group comprising polyvinyl alcohol-polyethylene glycol copolymers; polyvinyl alcohol or copolymers or mixtures thereof; polyvinylpyrrolidone-vinyl acetate copolymers; ethylcellulose dispersions; polyethylene glycol; polyvinyl alcohol and lecithin; polyvinylpyrrolidone; polymethylmetacrylate copolymers.

8. The solid oral pharmaceutical composition according to any of the preceding claims, wherein the composition further comprising at least one pharmaceutically acceptable excipient which is selected from a group comprising fillers, lubricants, disintegrants, glidants or mixtures thereof.

9. The solid oral pharmaceutical composition according to claim 1, wherein the weight ratio of microcrystalline cellulose to lactose is between 1.00 and 2.75.

10. The solid oral pharmaceutical composition according to claim 8, wherein the lubricant is selected from a group comprising sodium stearyl fumarate, magnesium stearate, calcium stearate, zinc stearate, talc, waxes, boric acid, hydrogenated vegetable oil, sodium chlorate, magnesium lauryl sulfate, sodium oleate, sodium acetate, sodium benzoate, polyethylene glycol, stearic acid, fatty acid, fumaric acid, glyceryl palmitostearate, sodium lauryl sulphate or mixtures thereof.

11. The solid oral pharmaceutical composition according to claim 10, wherein the lubricant is sodium stearyl fumarate.

12. The solid oral pharmaceutical composition according to claim 11, wherein the amount of sodium stearyl fumarate is between 1.00% and 10.00%, preferably between 3.00% and 5.00% by weight of the composition.

13. The solid oral pharmaceutical composition according to claim 12, wherein the weight ratio of sodium stearyl fumarate to microcrystalline cellulose is between 0.01 and 0.50, preferably between 0.02 and 0.20.

14. The pharmaceutical composition according to any of the preceding claims, comprising:
a. ivabradine hydrochloride 5.00%-10.00%
b. microcrystalline cellulose 40.00%-55.00%
c. lactose 20.00%-40.00%
d. croscarmellose sodium 5.00%-20.00%
e. colloidal silicon dioxide 0.50%-0.80%
f. sodium stearyl fumarate 3.00%-5.00%
g. coating 1.00%-5.00%

15. The pharmaceutical composition according to any of the preceding claims, comprising:
a. ivabradine hydrochloride 5.00%-10.00%
b. microcrystalline cellulose 40.00%-55.00%
c. lactose 20.00%-40.00%
d. sodium starch glycolate 5.00%-20.00%
e. colloidal silicon dioxide 0.50%-0.80%
f. sodium stearyl fumarate 3.00%-5.00%
g. coating 1.00%-5.00%

## Patentansprüche

1. Feste orale pharmazeutische Zusammensetzung, umfassend Ivabradinhydrochlorid und mikrokristalline Cellulose, wobei das Ivabradinhydrochlorid eine polymorphe Form aufweist, die aus der Gruppe ausgewählt ist, die aus Form S, Form II, Form IV, Form Delta und Form Delta D besteht; wobei die feste orale pharmazeutische Zusammensetzung ferner Lactose umfasst und das Gewichtsverhältnis von mikrokristalliner Cellulose zu Lactose größer als 1,00 ist.

2. Die feste orale pharmazeutische Zusammensetzung nach Anspruch 1, wobei die Zusammensetzung Ivabradinhydrochlorid in einer Menge von 0,01 bis 20,00 Gew.-% der Gesamtzusammensetzung, vorzugsweise 1,00 bis 10,00 Gew.-% und noch bevorzugter 5,00 bis 10,00 Gew.-% enthält.

3. Die feste orale pharmazeutische Zusammensetzung nach Anspruch 1, wobei die Menge an mikrokristalliner Cellulose zwischen 30,00 Gew.-% und 70,00 Gew.-% der Zusammensetzung, vorzugsweise zwischen 40,00 Gew.-% und 60,00 Gew.-%, noch bevorzugter zwischen 40,00 Gew.-% und 55,00 Gew.-% beträgt.

4. Die feste orale pharmazeutische Zusammensetzung gemäß Anspruch 1 oder 2, wobei die Zusammensetzung in Form einer beschichteten Tablette, einer dreischichtigen Tablette, einer zweischichtigen Tablette, einer mehrschichtigen Tablette, einer oral zerfallenden Tablette, einer Minitablette, Pellets, Zuckerpellets, Bukkaltablette, Sublingualtablette, Brausetablette, Tabletten mit sofortiger Freisetzung, Tabletten mit modifizierter Freisetzung, Filmtablette, magensaftlösliche Tablette, Pille, Kapsel, orales Granulat, Pulver, beschichtetes Perlen-System, Mikrosphäre, Tablette in Tablette, Einlagetablette, Dragee, Sachet oder oral verabreichbarer Film.

5. Die feste orale pharmazeutische Zusammensetzung gemäß Anspruch 4, wobei die Zusammensetzung in Form einer filmbeschichteten Tablette vorliegt.

6. Die feste orale pharmazeutische Zusammensetzung gemäß Anspruch 5, wobei die Filmbeschichtung eine Dicke von 1 bis 120 µm aufweist.

7. Die feste orale pharmazeutische Zusammensetzung nach Anspruch 6, wobei die Filmüberzug aus der Gruppe ausgewählt ist, die aus Polyvinylalkohol-Polyethylenglycol-Copolymeren, Polyvinylalkohol oder Copolymeren oder Mischungen davon, Polyvinylpyrrolidon-Vinylacetat-Copolymeren, Ethylcellulosedispersionen, Polyethylenglycol, Polyvinylalkohol und Lecithin, Polyvinylpyrrolidon, Polymethylmethacrylat-Copolymeren ausgewählt ist.

8. Die feste orale pharmazeutische Zusammensetzung nach einem der vorhergehenden Ansprüche, wobei die Zusammensetzung ferner mindestens einen pharmazeutisch verträglichen Hilfsstoff umfasst, der aus einer Gruppe ausgewählt ist, die Füllstoffe, Gleitmittel, Sprengmittel, Fließmittel oder Mischungen davon umfasst.

9. Die feste orale pharmazeutische Zusammensetzung nach Anspruch 1, wobei das Gewichtsverhältnis von mikrokristalliner Cellulose zu Lactose zwischen 1,00 und 2,75 liegt.

10. Die feste orale pharmazeutische Zusammensetzung gemäß Anspruch 8, wobei das Gleitmittel ausgewählt ist aus einer Gruppe, die Natriumstearylfumarat, Magnesiumstearat, Calciumstearat, Zinkstearat, Talk, Wachse, Borsäure, hydriertes Pflanzenöl, Natriumchlorat, Magnesiumsulfat, Natriumoleat, Natriumacetat, Natriumbenzoat, Polyethylenglykol, Stearinsäure, Fettsäure, Fumarsäure, Glycerylpalmitostearat, Natriumlaurylsulfat oder Mischungen davon ausgewählt ist.

11. Die feste orale pharmazeutische Zusammensetzung gemäß Anspruch 10, wobei das Gleitmittel Natriumstearylfumarat ist.

12. Die feste orale pharmazeutische Zusammensetzung nach Anspruch 11, wobei die Menge an Natriumstearylfumarat zwischen 1,00 % und 10,00 %, vorzugsweise zwischen 3,00 % und 5,00 Gew.-% der Zusammensetzung beträgt.

13. Feste orale pharmazeutische Zusammensetzung nach Anspruch 12, wobei das Gewichtsverhältnis von Natriumstearylfumarat zu mikrokristalliner Cellulose zwischen 0,01 und 0,50, vorzugsweise zwischen 0,02 und 0,20 liegt.

14. Die pharmazeutische Zusammensetzung gemäß einem der vorhergehenden nsprüche, umfassend:
a. Ivabradinhydrochlorid 5,00 %-10,00 %
b. mikrokristalliner Cellulose 40,00 %-55,00 %
c. Lactose 20,00 %-40,00 %
d. Croscarmellose-Natrium 5,00 %-20,00 %
e. kolloidales Siliciumdioxid 0,50 %-0,80 %
f. Natriumstearylfumarat 3,00 %-5,00 %
g. Überzug 1,00 %-5,00 %

15. Pharmazeutische Zusammensetzung nach einem der vorhergehenden Ansprüche, umfassend:
a. Ivabradinhydrochlorid 5,00 %-10,00 %
b. mikrokristalline Cellulose 40,00 %-55,00 %
c. Lactose 20,00 %-40,00 %
d. Natriumstärkeglycolat 5,00 %-20,00 %
e. kolloidales Siliciumdioxid 0,50 %-0,80 %
f. Natriumstearylfumarat 3,00 %-5,00 %
g. Überzug 1,00 %-5,00 %

## Revendications

1. - Composition pharmaceutique orale solide comprenant du chlorhydrate d'ivabradine et de la cellulose microcristalline, dans laquelle le chlorhydrate d'ivabradine a une forme polymorphe choisie dans le groupe consistant en la forme S, la forme II, la forme IV, la forme delta et la forme delta D ; dans laquelle ladite composition pharmaceutique orale comprend en outre du lactose et le rapport en poids de la cellulose microcristalline au lactose est supérieur à 1,00.

2. - Composition pharmaceutique orale solide selon la revendication 1, dans laquelle la composition comprend du chlorhydrate d'ivabradine dans une quantité de 0,01% à 20,00% en poids de la composition totale, de préférence de 1,00% à 10,00% et de façon davantage préférée de 5,00% à 10,00%.

3. - Composition pharmaceutique orale solide selon la revendication 1, dans laquelle la quantité de cellulose microcristalline est comprise entre 30,00 % et 70,00 % en poids de la composition, de préférence entre 40,00 % et 60,00 %, de façon davantage préférée entre 40,00 % et 55,00 %.

4. - Composition pharmaceutique orale solide selon l'une des revendications 1 ou 2, dans laquelle la composition se présente sous la forme de comprimés enrobés, comprimés tricouches, comprimés bicouches, comprimés multicouches, comprimés à désintégration orale, mini-comprimés, pastilles, pastilles de sucre, comprimés buccaux, comprimés sublinguaux, comprimés effervescents, comprimés à libération immédiate, comprimés à libération modifiée, comprimés pelliculés, comprimés à désingrégation gastrique, pilules, gélules, granulés oraux, poudre, système à perles enrobées, microsphères, formes de comprimé dans le comprimé, comprimés incrustés, dragées, sachets ou films administrables par voie orale.

5. - Composition pharmaceutique orale solide selon la revendication 4, dans laquelle la composition se présente sous la forme d'un comprimé pelliculé.

6. - Composition pharmaceutique orale solide selon la revendication 5, dans laquelle le pellicullage a une épaisseur de 1 à 120 µm.

7. - Composition pharmaceutique orale solide selon la revendication 6, dans laquelle le pellicullage est choisi dans le groupe comprenant les copolymères alcool polyvinylique-polyéthylène glycol ; l'alcool polyvinylique ou les copolymères ou mélanges de ceux-ci ; les copolymères polyvinylpyrrolidone-acétate de vinyle ; les dispersions d'éthylcellulose ; le polyéthylène glycol ; l'alcool polyvinylique et la lécithine ; la polyvinylpyrrolidone ; les copolymères de poly(méthacrylate de méthyle).

8. - Composition pharmaceutique orale solide selon l'une quelconque des revendications précédentes, dans laquelle la composition comprend en outre au moins un excipient pharmaceutiquement acceptable qui est choisi dans un groupe comprenant les charges, les lubrifiants, les désintégrants, les agents de glissement ou leurs mélanges.

9. - Composition pharmaceutique orale solide selon la revendication 1, dans laquelle le rapport pondéral de la cellulose microcristalline au lactose est entre 1,00 et 2,75.

10. - Composition pharmaceutique orale solide selon la revendication 8, dans laquelle le lubrifiant est choisi dans un groupe comprenant le fumarate de stéaryle et de sodium, le stéarate de magnésium, le stéarate de calcium, le stéarate de zinc, le talc, les cires, l'acide borique, l'huile végétale hydrogénée, le chlorate de sodium, le lauryl sulfate de magnésium, l'oléate de sodium, l'acétate de sodium, le benzoate de sodium, le polyéthylène glycol, l'acide stéarique, les acides gras, l'acide fumarique, le palmitostéarate de glycéryle, le lauryl sulfate de sodium ou leurs mélanges.

11. - Composition pharmaceutique orale solide selon la revendication 10, dans laquelle le lubrifiant est le fumarate de stéaryle et de sodium.

12. - Composition pharmaceutique orale solide selon la revendication 11, dans laquelle la quantité de fumarate de stéaryle et de sodium est entre 1,00 % et 10,00 %, de préférence entre 3,00 % et 5,00 % en poids de la composition.

13. - Composition pharmaceutique orale solide selon la revendication 12, dans laquelle le rapport en poids du fumarate de stéaryle et de sodium à la cellulose microcristalline est entre 0,01 et 0,50, de préférence entre 0,02 et 0,20.

14. - Composition pharmaceutique selon l'une quelconque des revendications précédentes, comprenant :
**a.** du chlorhydrate d'ivabradine 5,00%-10,00%
**b.** de la cellulose microcristalline 40,00%-55,00%
**c.** du lactose 20,00%-40,00%
**d.** de la croscarmellose et de sodium 5,00%-20,00%
**e.** du dioxyde de silicium colloïdal 0,50%-0,80%
f. du fumarate de stéaryle et de sodium 3,00%-5,00%
**g.** un enrobage 1,00%-5,00%

15. - Composition pharmaceutique selon l'une quelconque des revendications précédentes, comprenant :
**a.** du chlorhydrate d'ivabradine 5,00%-10,00%
**b.** de la cellulose microcristalline 40,00%-55,00%
**c.** du lactose 20,00%-40,00%
**d.** du glycolate d'amidon sodique 5,00%-20,00%
**e.** du dioxyde de silicium colloïdal 0,50%-0,80%
f. du fumarate de stéaryle et de sodium 3,00%-5,00%
**g.** un enrobage 1,00%-5,00%
